# EUROPÄISCHE PATENTSCHRIFT

(11) **EP 1 661 586 B1**
(45) Veröffentlichungstag und Bekanntmachung des Hinweises auf die Patenterteilung: **04.08.2010**
(21) Anmeldenummer: 05111116.9
(22) Anmeldetag: 23.11.2005
(51) Int. Cl.: A61L 2/18, C11D 3/395, C11D 17/04

(54) **Desinfizierendes Substrat**
Disinfecting substrate
Substrat désinfectant

(30) Priorität: 23.11.2004 DE 102004058143
(43) Veröffentlichungstag der Anmeldung: 31.05.2006
(73) Patentinhaber: Bode Chemie GmbH, 22525 Hamburg (DE)
(72) Erfinder: Bloß, Richard, 25462, Rellingen (DE); Meyer, Stephanie, 20535 Hamburg (DE); Fehling, Thomas, 20535 Hamburg (DE); Krug, Barbara, 20259, Hamburg (DE); Eckhardt, Christian, 22869 Schenefeld (DE); Dr. Pietsch, Hanns, 20148 Hamburg (DE)
(74) Vertreter: Oltmann, Eckhard

(56) Entgegenhaltungen:
- GB-A- 2 218 430
- US-A1- 2002 147 122
- US-A1- 2004 187 893
- US-B1- 6 511 950

## Beschreibung

Die vorliegende Erfindung betrifft ein mit einer antimikrobiellen Zubereitung getränktes Substrat zur Desinfektion von Oberflächen (Flächendesinfektion).

Die Desinfektion von Oberflächen spielt in vielen Bereichen des täglichen Lebens eine bedeutende Rolle, da der Kontakt von Mensch und Tier mit krankheitserregenden Mikroorganismen stets eine Gefahr für deren Gesundheit darstellt. In Krankenhäusern ist die Flächendesinfektion eine unverzichtbare Hygienemaßnahme. Die Anforderungen an die Präparate sind in den Richtlinien der DGHM festgelegt und auch in der europäischen Pränorm prEN 13713. Die Liste der von der DGHM gelisteten Flächendesinfektionsmittel enthält fast 500 (496) Präparate. Ein überwiegender Anteil davon basiert auf quaternären Ammoniumverbindungen (QAV) als mikrobiziden Wirkstoffen. Weitere für die Flächendesinfektion üblicherweise verwendeten Mikrobizide sind Aldehyde wie Formaldehyd, Glutaraldehyd oder Verbindungen welche diese Aldehyde abspalten. Hinzu kommen Amine und deren Derivate, Guanidine und in geringerem Maße noch Phenole oder Sauerstoffabspalter. Eindeutig die Spitzenposition nehmen jedoch die QAVs ein, häufig in Kombination untereinander, oder mit Aldehyden oder mit Aminen und selten mit Phenolen.

Neben der Auswahl des geeigneten Mittels ist auch das Verfahren der Desinfektion eine kritische Größe, die maßgeblich den Erfolg der Desinfektion bestimmt.
Üblicherweise werden Flächendesinfektionsmittel in Form von flüssigen wässrigen Lösungen angewendet, entweder als Konzentrat, welches vor Gebrauch verdünnt wird, oder als "ready to use"- Lösung, welche direkt verwendet werden kann. Zum Desinfizieren von Fußböden, Wänden oder anderen harten Oberflächen wird die Desinfektionslösung aus einem Eimer oder einem Vorratsgefäß mit einem Reinigungsutensil aufgenommen und auf die zu behandelnde Fläche verteilt (**"Eimermethode"**)

Eine besondere Produktform für Desinfektionsmittel stellen feste getränkte Substrate bzw. Textilien dar, insbesondere Tücher. Diese können bereits vom Hersteller mit der antimikrobiellen Zubereitung getränkt sein und haben dadurch den Vorteil, dass in ihnen die Zubereitung bereits in der richtigen Dosierung vorgegeben ist. Außerdem vermeiden sie den Nachteil von in Flaschen aufbewahrten Zubereitungen, deren Verpackung zerbrechen und deren Inhalt "auslaufen" kann.

Substrate in Form von Tüchern werden aus Textilien hergestellt. Textilien können gewebt, gestrickt oder gewirkt sein oder als Verbundstoff (engl. nonwoven textile) vorliegen. Meist werden (aus Kostengründen) Verbundstoffe verwendet. Bei Verbundstoffen erfolgt die Gewebebildung nicht durch Kette und Schuss oder Maschenbildung, sondern durch Verschlingung, und/oder kohäsive und/oder adhäsive Verbindung von Textilfasern. Verbundstoffe können nach der DIN 61210 T2 in Vlies, Papier Watte und Filz unterschieden werden. Vliese sind lockere Materialien aus Spinnfasern (d.h. Faser mit begrenzter Länge) oder Filamenten (Endlosfasern), deren Zusammenhalt im allgemeinen durch die den Fasern eigene Haftung gegeben ist. Hierbei können die Einzelfasern eine Vorzugsrichtung aufweisen (orientierte oder Kreuzlage-Vliese) oder ungerichtet (Wirrvliese) sein. Die Vliese können mechanisch verfestigt werden durch Vernadeln, Vermaschen oder durch Verwirbeln mittels scharfer Wasserstrahlen. Adhäsiv verfestigte Vliese entstehen durch Verkleben der Fasern mit flüssigen Bindemitteln (z.B. Acrylat-Polymere, SBR/NBR, Polyvinylester, Polyurethan-Dispersionen) oder durch Schmelzen oder Auflösen von sogenannten Bindefasern, die dem Vlies bei der Herstellung beigemischt wurden. Bei der kohäsiven Verfestigung werden die Faseroberflächen durch geeignete Chemikalien angelöst und durch Druck verbunden oder bei erhöhter Temperatur verschweißt [J. Falbe, M. Regnitz: Römpp-Chemie-Lexikon, 9. Aufl. Thieme-Verlag, Stuttgart (1992)].

Aus dem Stand der Technik sind außerdem Reinigungsartikel bekannt, welche faserhaltige Substrate umfassen : So offenbart US2002/147122 Reinigungsartikel aus porösem Material, welches bevorzugt aus olefinischen Polymerfasern gebildet wird. GB-A-2218430 offenbart eine Reinigungshilfe aus nicht gewebten Fasern, bevorzugt aus Polyester oder Polyamid. Der Polyester ist dort nicht näher charakterisiert.

Zur Flächendesinfektion werden überwiegend Tücher aus Baumwolle, Mikrofaser, Cellulose- oder modifizierter Cellulose verwendet.

All diese im Einsatz befindliche Tücher auf Basis von Cellulose oder modifizierter Cellulose haben den Nachteil, dass sie die in den Desinfektionslösungen enthaltenen, vorstehend genannten Wirkstoffe, besonders jedoch die quaternären Ammoniumverbindungen und Aminderivate zum Teil in erheblichem Maß adsorbieren. Folglich kann die Desinfektionsleistung nicht im vollen Umfang erbracht werden.

Diesem Nachteil versucht man nach dem Stande der Technik dadurch abzuhelfen, dass üblicherweise die Desinfektionslösung im Überschussverfahren mit Faserhaltigen Wischutensilien aufgetragen wird. Da Desinfektionsmittel jedoch relativ teuer sind und eine nicht unerhebliche toxikologische Belastung für den Anwender und die Umwelt (beispielsweise bei der "Entsorgung" der imprägnierten Textilfasern) darstellen, besteht ein erheblicher Bedarf, das Freisetzungsverhalten der antimikrobiellen Wirkstoffe von den Fasern zu verbessern.

Da neuerdings immer häufiger im "Unterschussverfahren" oder mit einer sogenannten "Sparmethode" gearbeitet wird, besteht die Gefahr, dass die Desinfektionsleistung nicht mehr in vollem Umfang erbracht wird.

Um Abhilfe zu schaffen, gibt es mittlerweile weitere bekannte Methoden, um die Nachteile der Adsorption von Wirkstoffen auf Fasern zu verringern. In der Patentschrift WO 2004/000373 und DE 10227872 wird deswegen eine Imprägnierung solcher Tücher mit qauternären Ammoniumverbindungen und Additiven beansprucht. In diesem Fall adsorbieren sie weniger Ammoniumverbindungen.

Der Nachteil ist ein zusätzlicher Arbeitsschritt zur Vorkonditionierung der Wischutensilien, verbunden mit erhöhten Kosten und Zeitbedarf. Wird das Verfahren nicht exakt eingehalten, so besteht die Gefahr einer unvollständigen Vorkonditionierung und möglicherweise Beeinflussung der Desinfektionswirkung. Außerdem bedeutet dieser zusätzliche Arbeitsschritt einen Mehrverbrauch von Wasser und Belastung der Umwelt durch die Konditioniermittel im Abwasser.

Es war nun die Aufgabe der vorliegenden Erfindung, die Nachteile des Standes der Technik zu beseitigen. Insbesondere war es die Aufgabe der vorliegenden Erfindung, ein einfach zu handhabendes Desinfektionssystem zu entwickeln, welches bei geringem Gehalt an desinfizierenden Substanzen wirkungsvoll zur Desinfektion (insbesondere zur Flächendesinfektion) geeignet ist.

Überraschend gelöst werden die Aufgaben durch ein desinfizierendes Substrat mit den Merkmalen des Anspruches 1.

Überraschend gelöst werden die Aufgaben ferner durch ein Verfahren zur Herstellung eines desinfizierenden Substrates, mit den Merkmalen des Anspruches 2.

Ferner werden die Aufgaben überraschend gelöst durch die Verwendung mit den Merkmalen des Anspruches 3.

Im Rahmen der vorliegenden Offenbarung wird unter erfindungsgemäß sowohl die erfindungsgemäßen Substrate als auch die nach dem erfindungsgemäßen Verfahren hergestellten Substrate sowie die erfindungsgemäß verwendeten Substrate verstanden.

Es hat sich jetzt überraschenderweise gezeigt, dass die Adsorption herkömmlicher Desinfektionsmittel, enthaltend quaternäre Ammoniumverbindungen durch die Verwendung von Kunststofftüchern aus speziellen Kunststoffmaterial deutlich verringert und in einigen Fällen ganz verhindert werden kann. Besonders ausgeprägt ist dieser Effekt wenn die Desinfektionslösungen quaternäre Ammoniumverbindungen enthalten, weil diese eine besonders starke Tendenz zum Aufziehen auf negativ geladene Oberflächen haben.

Überraschend und für den Fachmann nicht vorhersehbar war es insbesondere, dass die erfindungsgemäßen Substrate gewährleisten, dass die erfindungsgemäßen Zubereitungen (insbesondere flüssige Desinfektionsmittel) auf dem Trägermaterial ohne Adsorptionseffekte auf desinfizierende Wirkstoffe aufgetragen und bei der Anwendung wieder abgegeben werden können (siehe Abbildung 1 bis 3). Die erfindungsgemäßen Substrate haben gegenüber Substraten des Standes der Technik also den Vorteil, dass gerade die Wirkstoffe, die ein starkes Adsorptionsverhalten auf Fasern besitzen, bei diesem Verfahren nicht adsorbiert werden und bei Anwendung vom Substrat abgegeben werden können.

Das erfindungsgemäße Verfahren kann dabei erfindungsgemäß vorteilhaft zur Herstellung anwendungs- und "verkaufsfertiger" Substrate (die beispielsweise in Form von Tüchern, die in einer Sachet-Verpackung aufbewahrt und angeboten werden können) dienen. Erfindungsgemäß ist aber auch ein Verfahren, bei welchem die erfindungsgemäße Zubereitung getrennt von den erfindungsgemäßen Substraten angeboten und kurz vor der Anwendung mit diesem in Kontakt gebracht wird, eine Verfahrensweise, die beispielsweise von der Anwendung herkömmlicher Haushaltsreiniger her bekannt ist.

Das erfindungsgemäße Trägermaterial kann erfindungsgemäß vorteilhaft durch Eintauchen des Trägermaterials in ein Tauchbad, Ansprühen des Trägermaterials mit der Zubereitung oder durch Abstreifen Abstreifblechen, -balken oder -düsen des Trägermaterials an mit der Zubereitung absondernden Abstreifblechen, -balken oder -düsen imprägniert werden. Vorteilhafte Ausführungsformen der vorliegenden Erfindung sind dadurch gekennzeichnet, dass das gebrauchsfertige Trägermaterial mit 1 bis 10 Gramm Zubereitung pro Gramm Trägermaterial getränkt ist (Tränkungsgrad).

Die erfindungsgemäßen Trägermaterialien können gewebt, gestrickt oder gewirkt sein oder als Verbundstoff (engl. nonwoven textile) in Form von Vliesen, textilem Gewebe oder Schwämmen vorliegen. Bevorzugt werden (aus Kostengründen) Verbundstoffe verwendet. Die erfindungsgemäßen Trägermaterialien können mechanisch verfestigt werden durch Vernadeln, Vermaschen oder durch Verwirbeln mittels scharfer Wasserstrahlen. Zur besseren Aufnahme von Flüssigkeit können die Gewebe spezielle Ausführungsformen besitzen z.B. die Form von Frottiertücher aufweisen oder mit Noppen versehen sein.
Es ist erfindungsgemäß vorteilhaft, wenn das Trägermaterial in Form eines Tuches, eines Wischmops oder eines Schwammes vorliegt.

Erfindungsgemäß bevorzugt werden Trägermaterialien in Form von Vliesen und insbesondere in Form von Tüchern eingesetzt.

Erfindungsgemäße Trägermaterialien können Makroprägungen jeden gewünschten Musters oder Löcher/Öffnungen aufweisen.

Die Dicke eines erfindungsgemäßen Tuches ist hinsichtlich der "Nichtadsorption" unerheblich und ergibt sich aus dem Verfahren. Für "Einmal"-Tücher eignen sich besonders Vlies-Tücher mit einer Dicke von vorzugsweise 0,1 mm bis 5 mm. Die Wischtücher, welche bei der "Eimer"-Methode verwendet werden, können 5 mm bis 20 mm stark sein.

Erfindungsgemäß vorteilhafte antimikrobielle Wirkstoffe werden gewählt aus der Gruppe der Verbindungen Benzalkoniumchlorid, Didecyldimethylammoniumchlorid, Dioctyldimethylammoniumchlorid, Cetrimoniumbromid, Cetylpyridiniumchlorid (Hexadecylpyridiniumchlorid). Erfindungsgemäß bevorzugt ist dabei der Einsatz von Benzalkoniumchlorid.

Erfindungsgemäß vorteilhafte antimikrobielle Wirkstoffe aus der Gruppe der quartären Ammoniumverbindungen können gewählt werden aus der Gruppe Benzalkoniumchlorid, Didecyldimethylammoniumchlorid, Dioctyldimethylammoniumchlorid, Tetradecyldimethylethylammoniumethosulfat, Cetrimoniumbromid, Cetrimoniumchlorid, Cetylpyridiniumchlorid (Hexadecylpyridiniumchlorid), Didecylmethyl(oxyethyl)ammoniumpropionat, Decylisononyldimethylammoniumchlorid, Mecetroniumetilsulfat.

Erfindungsgemäß vorteilhafte antimikrobielle Wirkstoffe aus der Gruppe der Alkylamine können gewählt werden aus der Gruppe der Verbindungen N-(3-Aminopropyl)-N-dodecylpropan-1,3-diamin, N-Dodecylpropan-1.3-diamin, Alkylaminderivat.

Erfindungsgemäß vorteilhafte antimikrobielle Wirkstoffe aus der Gruppe der Guanidine können gewählt werden aus der Gruppe der Verbindungen Dodecylmethylguanidinacetat, Dodecylguanidinacetat, Octadecylguanidinacetat, Polyhexamethylenbiguanid-Hydrochlorid, Cocospropylendiamin-1,5-bisguanidiniumacetat.

Erfindungsgemäß vorteilhafte antimikrobielle Wirkstoffe aus der Gruppe der Aldehyde (wozu erfindungsgemäß auch Formaldehydabspalter gezählt werden) sind Formaldehyd, Glutaraldehyd, Glutaraldehydabspalter, Glyoxal, Succindialdehyd, o-Phthaldialdehyd.

Erfindungsgemäß vorteilhafte antimikrobielle Wirkstoffe aus der Gruppe der Phenole sind o-, m-, p-Kresol, p-Chlor-m-kresol, p-Chlor-m-xylenol, Isopropyl-o-kresol, 4-Isopropyl-m-kresol, o-Phenylphenol, 4-Chlorthymol.

Erfindungsgemäß vorteilhafte antimikrobielle Wirkstoffe aus der Gruppe der Sauerstoffabspalter sind Peressigsäure, Peressigsäureabspalter, Percarbonat, Persulfat, Perphthalat.

Erfindungsgemäß vorteilhafte antimikrobielle Wirkstoffe aus der Gruppe der Alkohole sind Phenoxyethanol, Phenoxypropanol.

Erfindungsgemäß besonders bevorzugt ist der Einsatz von Benzalkoniumchlorid, Didecylmethyl(oxyethyl)ammoniumpropionat und Didecyldimethylammoniumchlorid als antimikrobiellem Wirkstoff.

Erfindungsgemäß vorteilhaft liegt die erfindungsgemäße Zubereitung in Form einer wässrigen oder wässrig-alkoholischen Lösung vor. Im letzteren Falle enthält die Zubereitung die antimikrobiell wirkenden Alkohole Ethylakolhol, Isopropylakohol und/oder n-Propanol als Lösungsmittelbestandteil, die nicht zu den erfindungsgemäßen Wirkstoffen zugerechnet werden.

Die Einsatzkonzentrationen der erfindungsgemäßen Wirkstoffe werden erfindungsgemäß vorteilhaft (ebenso wie die Einwirkungszeiten bei der erfindungsgemäßen Anwendung) in der Zubereitung entsprechend den Vorgaben der Desinfektionsmittel-Kommission der Deutsches Gesellschaft für Hygiene und Mikrobiologie, veröffentlicht im mhp Verlag GmbH, Wiesbaden 2002 bzw. 2004) und den Vorgaben der Kommission für Krankenhaushygiene beim Robert-Koch-Institut (Bundesgesundheitsblatt-Gesundheitsforschung-Gesundheitsschutz, 2004, 47; 51-61) gewählt.

Bei wässrigen Zubereitungen liegt der Wirkstoffgehalt bei der Anwendung der Zubereitung erfindungsgemäß bevorzugt bei 0,01 bis 1 Gewichts-%, bezogen auf das Gesamtgewicht der Zubereitung.

Bei alkoholischen Produkten ist eine Anwendungskonzentration von 10 bis 80 Gewichts-% Alkohol, bezogen auf das Gesamtgewicht der Zubereitung, erfindungsgemäß besonders vorteilhaft.

Generell ist ein Wirkstoffgehalt von mindestens 0,5 Gewichts-%, bezogen auf das Gesamtgewicht der Zubereitung, besonders vorteilhaft.

Erfindungsgemäß vorteilhaft können die erfindungsgemäßen Zubereitungen weitere Inhaltsstoffe wie Tenside, Schaumregulatoren, Komplexbildner, Duftstoffe, pH-Wert-Regulatoren, Verdickungsmittel etc. enthalten, wie sie dem Fachmann für Desinfektionsmittel aus dem Stande der Technik her geläufig sind.

Vorzugsweise können die Zubereitungen nichtionische Tenside enthalten, ausgewählt aus der Liste der Alkylethoxylate und/oder und/oder Phenylakylethoxylate und/oder Alkylpropoxylate, deren Alkylgruppe eine gesättigte oder ungesättigte, gerad- oder verzweigtkettige Alkylgruppe mit 8 bis 18, vorzugsweise 12 bis 14 Kohlenstoffatomen ist.
Dabei ist es erfindungsgemäß bevorzugt, wenn diese Tenside in einer Gesamtkonzentration von 1 bis 15 Gew.-%, bezogen auf das Gesamtgewicht der Zubereitung, in der Zubereitung enthalten sind.

Vorzugsweise können die Zubereitungen Komplexbildner enthalten, ausgewählt aus der Liste der Carbonsäurederivate und deren Salze wie Ethylendiamintetraessigsäure und/oder Nitrilotriessigsäure und/oder Methylglycindiessigsäure und/oder Diethylentriaminpentaessigsäure und der Liste der Phosphonsäuren und deren Salze wie Hydroxyethan-di-phosphonsäure und/oder Amino-tris-methylenphosphonsäure und/oder Diethylentriamin-penta-methylenphosphonsäure und/oder Hydroxyethylamono-dimethylenphosphonsäure.
Dabei ist es erfindungsgemäß bevorzugt, wenn diese Komplexbildner in einer Gesamtkonzentration von 1 bis 5 Gew.-%, bezogen auf das Gesamtgewicht der Zubereitung, in der Zubereitung enthalten sind.

Erfindungsgemäß vorteilhaft kann die erfindungsgemäße Zubereitung einen oder mehrere Schaumregulatoren enthalten, die insbesondere vorteilhaft gewählt werden können aus der Gruppe der Verbindungen der Glykole, Alkylisononanamide und Ethoxylate, Silikone, Polysiloxane, Fettsäurederivate, Phosphonsäureester.

Die Zubereitungen können entweder als Konzentrate, die vor Gebrauch verdünnt werden oder als "ready to use" Fertig-Lösungen mit dem erfindungsgemäßen Träger auf die zu behandelnde Fläche aufgebracht werden.

Die erfindungsgemäßen Substrate finden erfindungsgemäß vorteilhaft Verwendung zur Reinigung und Desinfektion von Oberflächen, insbesondere von harten Oberflächen.

In einer speziellen Anwendung liegt die entsprechende Tuchqualität in Form einer Rolle in einem Vorratsbehälter wie z. B. einer Dose oder einem Eimer zusammen mit einer definierten Menge des Desinfektionsmittels in der Anwendungskonzentration.

### Vergleichsversuche

Als Desinfektionsmittel wurde ein aldehydfreies Produkt gewählt, bei dem zu erwarten war, dass ein Teil des Wirkstoffes auf den Fasern adsorbiert. Der Behälter wurde danach fest verschlossen, die Tücher konnten sich nun vollständig mit der Desinfektionsmittellösung benetzen.
Über einen definierten Zeitraum wurden regelmäßig Tücher entnommen um eine praxisnahe Vorgehensweise zu gewährleisten.

Während dieses Zeitraums erfolgte regelmäßig eine Probennahme mit anschließender Wirkstoffbestimmung aus der Desinfektionsmittellösung der ausgepressten Tücher.

### Verwendete Wischtücher:

| | Naturviskosefaser 100 % | Polyesterverstärkter Zellstoff | Polyestervlies (PET) |
|---|---|---|---|
| Tuchgröße | ca. 20 x 38 cm | ca. 20 x 38 cm | ca. 20 x 38 cm |
| Gewicht | ca: 75 g/m² | ca. 60 g/m² | ca. 70 g/m² |

### 1) Beispiel1 (Vergleichsbeispiel) :

Eine Rolle mit reiner Naturfaser aus 100% Naturviskosefaser mit 90 Tüchern mit den Abmessungen 20 x 38 cm/Tuch wurde mit 3l einer anwendungsfertigen Desinfektionslösung, welche den Wirkstoff Benzalkoniumchlorid enthielt, getränkt und in einen Vorratsbehälter gelegt, der fest verschlossen wurde. Nachdem die Rolle vollständig benetzt war, wurden in regelmäßigen Abständen Tücher entnommen, ausgepresst und aus der ausgepressten Lösung der Wirkstoffgehalt analysiert. Das Ergebnis ist in Abbildung 1 dargestellt, indem die Konzentration der Desinfektionslösung angegeben wird. Bereits nach 3 Tagen erfolgt eine Abnahme um 50 - 60%.

### Abbildung 1: Beispiel einer Adsoptionskurve:

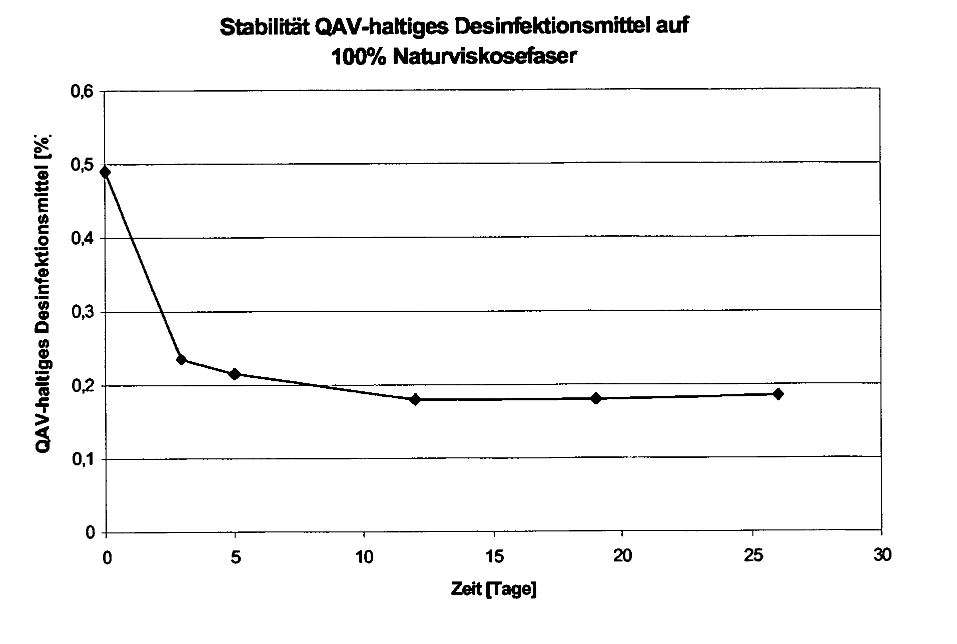

### 2) Beispiel 2 (Vergleichsbeispiel):

In einem Vergleichsversuch wurde mit der gleichen Desinfektionslösung ein Vlies aus polyesterverstärktem Zellstoff getränkt und wie in Beispiel 1 verfahren. Das Ergebnis ist in Abbildung 2 dargestellt. Bereits nach 3 Tagen erfolgt eine drastische Konzentrationsabnahme um mehr als 60%.

### Abbildung 2 Beispiel einer Adsoptionskurve mit Mischung Naturfaser/Kunstfaser

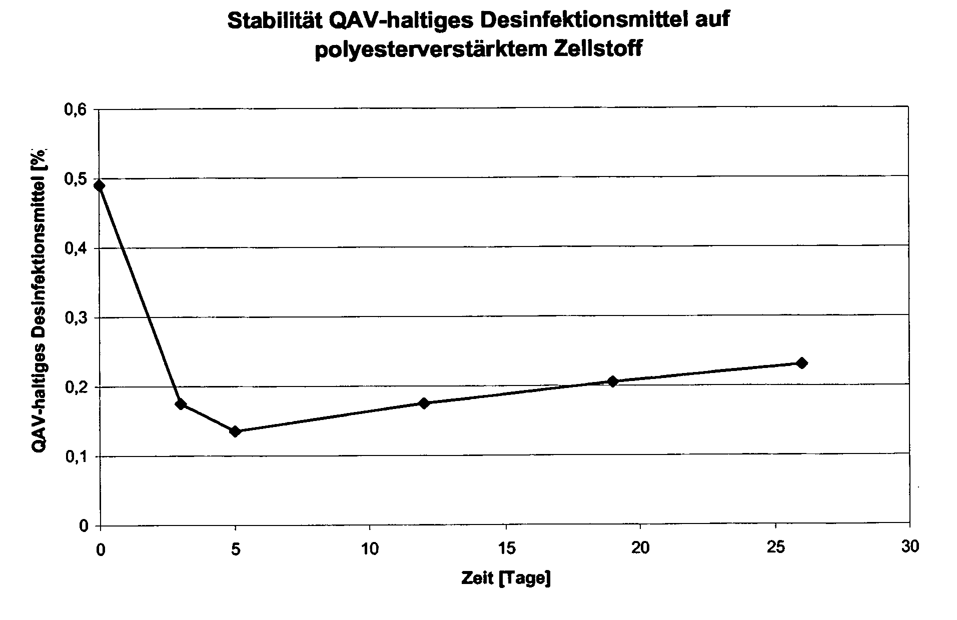

### 3) Beispiel 3 (Verwendung der erfindungsgemäßen Tücher):

In einem Vergleichsversuch mit den erfindungsgemäßen Tüchern wurde mit der gleichen Desinfektionslösung ein Tuch aus Polyestervlies getränkt und wie in Beispiel 1 verfahren. Das Ergebnis ist in Abbildung 3 dargestellt. Es erfolgt keine Adsorption bei Verwendung der erfindungsgemäßen Tücher.

### Abbildung 3 Beispiel einer Adsoptionskurve mit dem erfindungsgemäßen Vlies Polyester:

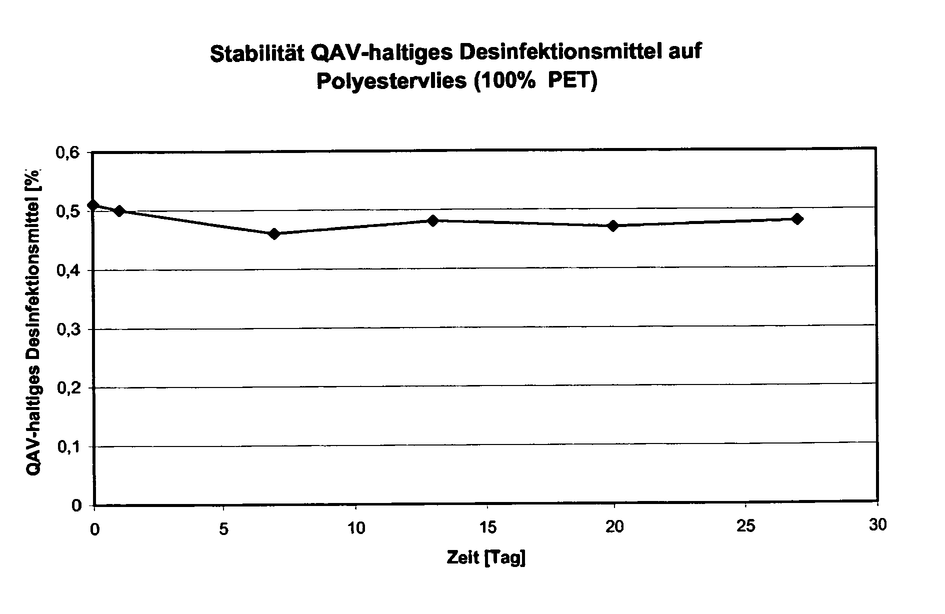

Die nachfolgenden Beispiele stellen erfindungsgemäß bevorzugte Ausführungsformen der vorliegenden Erfindung dar und sollen die vorliegende Erfindung verdeutlichen ohne sie einzuschränken.

### Beispielrezepturen von Desinfektionsmitteln (abgekürzt mit DM):

| Inhaltsstoffe | DM 1 | DM 2 | DM 3 ready to use | DM 4 | DM 5 | DM 6 |
|---|---|---|---|---|---|---|
| Benzalkoniumchlorid | 19,9 % | 3 % | -- | 13 % | 20 % | -- |
| Didecyldimethylammoniumchlorid | -- | 3 % | -- | -- | -- | -- |
| Guanidiniumderivat | -- | -- | -- | 9,2 % | -- | -- |
| Mecetroniumetilsulfat | -- | -- | 0,04 % | -- | -- | -- |
| N-(3-Aminopropyl)-N-dodecylpropan-1,3-diamin | 5 % | -- | -- | -- | -- | -- |
| Alkylaminderivat | -- | -- | -- | -- | -- | 26 % |
| (Ethylendioxy)-dimethanol | -- | -- | 0,19 % | -- | -- | -- |
| Glutaral | -- | 5 % | -- | -- | -- | -- |
| Phenoxypropanol | -- | -- | -- | -- | 35 % | -- |
| Phenoxyethanol | -- | -- | -- | -- | -- | 1 - 15 % |
| Ethanol | -- | -- | 32,3 % | -- | -- | -- |
| Propan-2-ol | -- | -- | 3 % | 1 - 10 % | 1 -10 % | --- |
| Propan-1-ol | -- | -- | 11 % | -- | -- | -- |
| Nichtionisches Tensid | 1 - 15 % | 1 - 15 % | -- | 1 - 15 % | 1 -15 % | 1 - 15 % |
| Komplexbildner | -- | -- | -- | -- | 1 - 5 % | 1 - 5 % |
| Schaumregulatoren | 0 - 1 % | -- | -- | 0 - 1 % | 0 - 1 % | 0 - 1 % |
| Lösungsmittel | 0 - 10 % | -- | -- | 0 - 10 % | -- | 0 - 15 % |
| pH-Regulator | 0 - 5% | 0 - 5% | -- | 0 - 5% | 0 - 5% | 0 - 5% |
| Duftstoffe | 0 - 1 % | 0 - 1 % | 0 - 1% | 0 - 1% | 0 - 1% | 0 - 1% |
| Wasser | ad 100 % | ad 100 % | ad 100 % | ad 100 % | ad 100 % | ad 100 % |

Der Tränkungsgrad beträgt ca. 6g Desinfektionsmittellösung / g Tuch (Polyestervlies 100 % PET).

## Patentansprüche

1. Desinfizierendes Substrat, welches gebildet wird aus
a) einem Trägermaterial aus Polyethylenterephthalat (PET),
b) einer Zubereitung enthaltend einen oder mehrere antimikrobielle Wirkstoffe gewählt aus der Gruppe der Verbindungen der quaternären Ammoniumverbindungen, Guanidine, Alkylamine und/oder deren Derivate, Aldehyde und/oder Phenole.

2. Verfahren zur Herstellung eines desinfizierenden Substrates, **dadurch gekennzeichnet, dass** ein Trägermaterial aus Polyethylenterephthalat (PET) mit einer Zubereitung enthaltend einen oder mehrere antimikrobielle Wirkstoffe gewählt aus der Gruppe der Verbindungen der quaternären Ammoniumverbindungen, Guanidine, Alkylamine und/oder deren Derivate, Aldehyde und/oder Phenole getränkt und/oder benetzt wird.

3. Verwendung eines Trägermaterials aus Polyethylenterephthalat (PET) zur Auftragung oder Verteilung einer Zubereitung enthaltend einen oder mehrere antimikrobielle Wirkstoffe gewählt aus der Gruppe der Verbindungen der quaternären Ammoniumverbindungen, Guanidine, Alkylamine und/oder deren Derivate, Aldehyde und/oder Phenole, ohne dass es zu einer Verringerung der Wirksamkeit der antimikrobiellen Zubereitung durch Adsorbtion der Wirkstoffe an das Trägermaterial kommt.

4. Substrat, Verwendung oder Verfahren nach einem der vorhergehenden Ansprüche, **dadurch gekennzeichnet, dass** das Trägermaterial in Form von Vlies, textilem Gewebe, eines Tuches, eines Wischmops oder eines Schwammes vorliegt.

5. Substrat, Verwendung oder Verfahren nach einem der vorhergehenden Ansprüche, **dadurch gekennzeichnet, dass** einer oder mehrere antimikrobielle Wirkstoffe gewählt werden aus der Gruppe Benzalkoniumchlorid, Didecyldimethylammoniumchlorid, Dioctyldimethylammoniumchlorid, Tetradecyldimethylethylammoniumethosulfat, Cetrimoniumbromid, Cetrimoniumchlorid, Cetylpyridiniumchlorid (Hexadecylpyridiniumchlorid), Didecylmethyl(oxyethyl)ammoniumpropionat, Decylisononyldimethylammoniumchlorid, Mecetroniumetilsulfat, N-(3-Aminopropyl)-N-dodecylpropan-1,3-diamin, N-Dodecylpropan-1.3-diamin, Alkylaminderivat, Dodecylmethylguanidinacetat, Dodecylguanidinacetat, Octadecylguanidinacetat, Polyhexamethylenbiguanid-Hydrochlorid, Cocospropylendiamin-1,5-bisguanidiniumacetat, Formaldehyd, Formaldehydabspalter, Glutaraldehyd, Glutaraldehydabspalter, Glyoxal, Succindialdehyd, o-Phthaldialdehyd, o-, m-, p-Kresol, p-Chlor-m-kresol, p-Chlor-m-xylenol, Isopropyl-o-kresol, 4-Isopropyl-m-kresol, o-Phenylphenol, 4-Chlorthymol, Phenoxyethanol, Phenoxypropanol, Peressigsäure, Peressigsäureabspalter, Percarbonat, Persulfat, Perphthalat.

6. Substrat, Verwendung oder Verfahren nach einem der vorhergehenden Ansprüche **dadurch gekennzeichnet, dass** die Zubereitung (Wirkstofflösung) in Form einer wässrigen oder wässrig-alkoholischen Lösung vorliegt.

7. Substrat, Verwendung oder Verfahren nach einem der vorhergehenden Ansprüche zur Verwendung für die Reinigung und Desinfektion von harten Oberflächen.

8. Substrat, Verwendung oder Verfahren nach einem der vorhergehenden Ansprüche, **dadurch gekennzeichnet, dass** das gebrauchsfertige Trägermaterial mit 1 bis 10 Gramm Zubereitung pro Gramm Trägermaterial getränkt ist (Tränkungsgrad).

9. Substrat, Verwendung oder Verfahren nach einem der vorhergehenden Ansprüche, **dadurch gekennzeichnet, dass** die Zubereitung Tenside, Schaumregulatoren, Komplexbildner, Duftstoffe, pH-Wert-Regulatoren und/oder Verdickungsmittel enthält.

## Claims

1. Disinfecting substrate which is formed from
a) a carrier material made of polyethylene terephthalate (PET),
b) a preparation comprising one or more antimicrobial active ingredients selected from the group of the compounds of the quaternary ammonium compounds, guanidines, alkylamines and/or derivatives thereof, aldehydes and/or phenols.

2. Method for producing a disinfecting substrate, **characterized in that** a carrier material made of polyethylene terephthalate (PET) is impregnated and/or wetted with a preparation comprising one or more antimicrobial active ingredients selected from the group of the compounds of the quaternary ammonium compounds, guanidines, alkylamines and/or derivatives thereof, aldehydes and/or phenols.

3. Use of a carrier material made of polyethylene terephthalate (PET) for the application or distribution of a preparation comprising one or more antimicrobial active ingredients selected from the group of the compounds of the quaternary ammonium compounds, guanidines, alkylamines and/or derivatives thereof, aldehydes and/or phenols, without resulting in a reduction in the effectiveness of the antimicrobial preparation as a result of adsorption of the active ingredients onto the carrier material.

4. Substrate, use or method according to one of the preceding claims, **characterized in that** the carrier material is in the form of nonwoven, textile fabric, a wipe, a mop or a sponge.

5. Substrate, use or method according to one of the preceding claims, **characterized in that** one or more antimicrobial active ingredients are selected from the group consisting of benzalkonium chloride, didecyldimethylammonium chloride, dioctyldimethylammonium chloride, tetradecyldimethylethylammonium methosulphate, cetrimonium bromide, cetrimonium chloride, cetylpyridinium chloride (hexadecylpyridinium chloride), didecylmethyl(oxyethyl)ammonium propionate, decylisononyldimethylammonium chloride, mecetronium metilsulphate, N-(3-aminopropyl)-N-dodecylpropane-1,3-diamine, N-dodecylpropane-1,3-diamine, alkylamine derivative, dodecylmethylguanidine acetate, dodecylguanidine acetate, octadecylguanidine acetate, polyhexamethylene biguanide hydrochloride, cocopropylenediamine-1,5-bisguanidinium acetate, formaldehyde, formaldehyde donor, glutaraldehyde, glutaraldehyde donor, glyoxal, succindialdehyde, o-phthaldialdehyde, o-, m-, p-cresol, p-chloro-m-cresol, p-chloro-m-xylenol, isopropyl-o-cresol, 4-isopropyl-m-cresol, o-phenylphenol, 4-chlorothymol, phenoxyethanol, phenoxypropanol, peracetic acid, peracetic acid donor, percarbonate, persulphate, perphthalate.

6. Substrate, use or method according to one of the preceding claims, **characterized in that** the preparation (active ingredient solution) is in the form of an aqueous or aqueous-alcoholic solution.

7. Substrate, use or method according to one of the preceding claims for use for the cleaning and disinfection of hard surfaces.

8. Substrate, use or method according to one of the preceding claims, **characterized in that** the ready-to-use carrier material is impregnated with 1 to 10 grams of preparation per gram of carrier material (degree of impregnation).

9. Substrate, use or method according to one of the preceding claims, **characterized in that** the preparation comprises surfactants, foam regulators, complexing agents, fragrances, pH regulators and/or thickeners.

## Revendications

1. Substrat désinfectant, qui est formé par
a) un matériau support en poly(téréphtalate d'éthylène) (PET),
b) une composition contenant une ou plusieurs substances actives antimicrobiennes choisies dans le groupe des composés formé par les composés d'ammonium quaternaire, les guanidines, les alkylamines et/ou leurs dérivés, les aldéhydes et/ou les phénols.

2. Procédé pour la préparation d'un substrat désinfectant, **caractérisé en ce qu'**un matériau support en poly(téréphtalate d'éthylène) (PET) est imprégné et/ou mouillé avec une composition contenant une ou plusieurs substances actives antimicrobiennes choisies dans le groupe des composés formé par les composés d'ammonium quaternaire, les guanidines, les alkylamines et/ou leurs dérivés, les aldéhydes et/ou les phénols.

3. Utilisation d'un matériau support en poly(téréphtalate d'éthylène) (PET) pour l'application ou la répartition d'une composition contenant une ou plusieurs substances actives antimicrobiennes choisies dans le groupe des composés formé par les composés d'ammonium quaternaire, les guanidines, les alkylamines et/ou leurs dérivés, les aldéhydes et/ou les phénols, sans qu'il ne se produise une diminution de l'efficacité de la composition antimicrobienne par adsorption des substances actives sur le matériau support.

4. Substrat, utilisation ou procédé selon l'une quelconque des revendications précédentes, **caractérisé**(e) en ce que le matériau support se trouve sous forme de non-tissé, de tissu textile, de drap, de balai à franges ou d'éponge.

5. Substrat, utilisation ou procédé selon l'une quelconque des revendications précédentes, **caractérisé**(e) en ce qu'une ou plusieurs substances actives antimicrobiennes sont choisies dans le groupe formé par le chlorure de benzalconium, le chlorure de didécyldiméthylammonium, le chlorure de dioctyldiméthylammonium, l'éthosulfate de tétradécyldiméthyléthylammonium, le bromure de cétrimonium, le chlorure de cétrimonium, le chlorure de cétylpyridinium (chlorure d'hexadécylpyridinium), le propionate de didécylméthyl(oxyéthyl)ammonium, le chlorure de décylisononyldiméthylammonium, l'étilsulfate de mécétronium, la N-(3-aminopropyl)-N-dodécylpropane-1,3-diamine, la N-dodécylpropane-1,3-diamine, les dérivés d'alkylamine, l'acétate de dodécylméthylguanidine, l'acétate de dodécylguanidine, l'acétate d'octadécylguanidine, le chlorhydrate de polyhexaméthylènebiguanide, l'acétate de cocopropylènediamine-1,5-bisguanidinium, le formaldéhyde, les agents de dissociation du formaldéhyde, le glutaraldéhyde, les agents de dissociation du glutaraldéhyde, le glyoxal, le dialdéhyde succinique, le dialdéhyde o-phtalique, l'o-crésol, le m-crésol, le p-crésol, le p-chloro-m-crésol, le p-chloro-m-xylénol, l'isopropyl-o-crésol, le 4-isopropyl-m-crésol, l'o-phénylphénol, le 4-chlorothymol, le phénoxyéthanol, le phénoxypropanol, l'acide peracétique, les agents de dissociation de l'acide peracétique, le percarbonate, le persulfate, le perphtalate.

6. Substrat, utilisation ou procédé selon l'une quelconque des revendications précédentes, **caractérisé**(e) en ce que la composition (solution de substance active) se trouve sous forme de solution aqueuse ou aqueuse-alcoolique.

7. Substrat, utilisation ou procédé selon l'une quelconque des revendications précédentes destiné(e) à une utilisation pour la purification et la désinfection de surfaces dures.

8. Substrat, utilisation ou procédé selon l'une quelconque des revendications précédentes, **caractérisé**(e) en ce que le matériau support prêt à l'emploi est imprégné avec 1 à 10 grammes de composition par gramme de matériau support (degré d'imprégnation).

9. Substrat, utilisation ou procédé selon l'une quelconque des revendications précédentes, **caractérisé**(e) en ce que la composition contient des agents tensioactifs, des régulateurs de mousse, des complexants, des parfums, des régulateurs de pH et/ou des épaississants.
